# EUROPÄISCHE PATENTSCHRIFT

(11) **EP 2 736 459 B1**
(45) Veröffentlichungstag und Bekanntmachung des Hinweises auf die Patenterteilung: **28.06.2017**
(21) Anmeldenummer: 12755947.4
(22) Anmeldetag: 27.07.2012
(51) Int. Cl.: A61F 2/954, A61F 2/958, A61F 2/97

(54) **VORRICHTUNG ZUR VORGEBBAREN ANORDNUNG EINES STENTS**
DEVICE FOR THE PREDETERMINABLE ARRANGEMENT OF A STENT
DISPOSITIF POUR LA DISPOSITION PRÉDÉFINISSABLE D'UN STENT

(30) Priorität: 29.07.2011 AT 11082011
(43) Veröffentlichungstag der Anmeldung: 04.06.2014
(73) Patentinhaber: Gaul, Georg, 1190 Wien (AT)
(72) Erfinder: Gaul, Georg, 1190 Wien (AT)
(74) Vertreter: Gibler & Poth Patentanwälte KG
(86) Internationale Anmeldenummer: PCT/AT2012/000199
(87) Internationale Veröffentlichungsnummer: WO 2013/016749

(56) Entgegenhaltungen:
- EP-A1- 2 036 519
- WO-A1-97/15346
- WO-A1-2008/157760
- WO-A1-2009/035957
- US-A1- 2005 021 070
- US-A1- 2006 030 924
- US-A1- 2008 033 525

## Beschreibung

Die Erfindung betrifft eine Vorrichtung zur vorgebbaren Anordnung eines Stents in einem Blutgefäß gemäß dem Oberbegriff des Patentanspruches 1.

Ein Stent ist eine scherengitterartige Gefäßstütze, welche dazu dient, Einengungen von Blutgefäßen oder anderen Lumen des Körpers dauerhaft zu erweitern. Solche Einengungen können beispielsweise krankheitsbedingt sein oder durch Ablagerungen an den Gefäßwänden hervorgerufen werden. Der Stent wird hierfür mittels eines Ballonkatheters durch einen arteriellen Zugang zu der gewünschten Stelle transportiert und dann durch ein Aufblasen dieses Ballons, daher einer Battonditatation, an der gewünschten Stelle gegen das Gefäß gepresst und damit fixiert.

Eine große Herausforderung stellen hierbei sogenannte Bifurkationen, daher Verzweigungen der Blutgefäße dar. Bei einer Bifurkation zweigt von einem Hauptast des Blutgefäßes ein Nebenast ab. Als Fachausdruck wird für den Hauptast oft das englische Wort "main branch", und für den Nebenast das englische Wort "side branch" verwendet. Der Abgang des Nebenastes vom Hauptast wird als Ostium bezeichnet. Hierbei werden in der Regel zwei Stents gesetzt, einer im Hauptast und einer im Nebenast, wobei der Stent im Nebenast in der Regel über das Ostium hinaus in den Hauptast des Blutgefäßes ragt

Nachteilig daran ist, dass der in den Hauptast ragende Teil des Stents des Nebenastes zu Komplikationen führen kann.

Die US 2008/033525 A1 beschreibt ein Kathetersystem, bei welchem zwei seriell hintereinander angeordnete Ballone vorgesehen sind.

Die WO 97/15346 A1 offenbart eine Anordnung aus zwei separaten Ballonen, welche parallel nebeneinander liegen, und welche an einer Bifurkation jeweils nebeneinander in unterschiedliche Blutgefäße eindringen sollen.

Die EP 2 036 519 A1 und die US 2006/0030924 A1 beschreiben ebenfalls jeweils Vorrichtungen mit zwei nebeneinender angeordneten Ballonen.

Aus der WO 2008/157760 A1 geht ein weiteres Ballonkathetersystem hervor.

Die US 2005/021070 A1 beschreibt ein Kathetersystem mit einem dünnen Ballon und einem dünnen Stent, welche dazu ausgebildet sind, durch die Maschen eines, in einem Hauptast angeordneten Stents in einen Nebenast geschoben zu werden. Aus WO 2009/035957 A1 ist ein Ballonkatheter bekannt mit einem Set von zwei Ballons mit unterschiedlicher Expansionsfähigkeit. Aufgabe der Erfindung ist es daher eine Vorrichtung der eingangs genannten Art anzugeben, mit welcher die genannten Nachteile vermieden werden können, mit welcher die eingesetzten Stents an die Form der Bifurkation angepasst werden können.

Erfingdungsgemäß wird dies durch die Merkmale des Patentanspruches 1 erreicht. Dadurch ergibt sich der Vorteil, dass die Einengung an der Bifurkation vollständig beseitigt werden kann. Dabei kann der Stent des Nebenast vollständig an die Gefäßwand des Hauptastes im Bereich des Ostiums angelegt werden, wodurch ein stabiler Zusammenhalt der Gefäßwand des Hauptastes mit dem Stent des Nebenastes erreicht werden kann. Weiters wird dadurch verhindert, dass Teile des Stents des Nebenastes in den Hauptast hineinragen und zu Komplikationen führen. Das vollständige Anlegen des Stents des Nebenastes an das Ostium kann mit einem Schritt erfolgen, wodurch die Zeit des medizinischen Eingriffes kurz gehalten werden kann, wodurch die Sicherheit des Patienten erhöht werden kann.

Die Unteransprüche betreffen weitere vorteilhafte Ausgestaltungen der Erfindung.

Ausdrücklich wird hiermit auf den Wortlaut der Ansprüche Bezug genommen, wodurch die Ansprüche an dieser Stelle durch Bezugnahme in die Beschreibung eingefügt sind und als wörtlich wiedergegeben gelten.

Die Erfindung wird unter Bezugnahme auf die beigeschlossenen Zeichnungen, in welchen lediglich bevorzugte Ausführungsformen beispielhaft dargestellt sind, näher beschrieben. Dabei zeigt:
Fig. 1 eine erste bevorzugte Ausführungsform der Vorrichtung im schlaffen Zustand im Blutgefäß;
Fig. 2 die erste bevorzugte Ausführungsform der Vorrichtung im aufgeblasenen Zustand im Blutgefäß;
Fig. 3 die erste bevorzugte Ausführungsform der Vorrichtung im schlaffen Zustand;
Fig. 4 die erste bevorzugte Ausführungsform der Vorrichtung im aufgeblasenen Zustand;
Fig. 5 eine zweite bevorzugte Ausführungsform der Vorrichtung im schlaffen Zustand;
Fig. 6 die zweite bevorzugte Ausführungsform der Vorrichtung im aufgeblasenen Zustand;
Fig. 7 eine dritte bevorzugte Ausführungsform der Vorrichtung im schlaffen Zustand;
Fig. 8 die dritte bevorzugte Ausführungsform der Vorrichtung im aufgeblasenen Zustand;
Fig. 9 eine vierte bevorzugte Ausführungsform der Vorrichtung im schlaffen Zustand als Schnitt;
Fig. 10 die vierte bevorzugte Ausführungsform der Vorrichtung im halb aufgeblasenen Zustand; und
Fig. 11 die vierte bevorzugte Ausführungsform der Vorrichtung im aufgeblasenen Zustand.

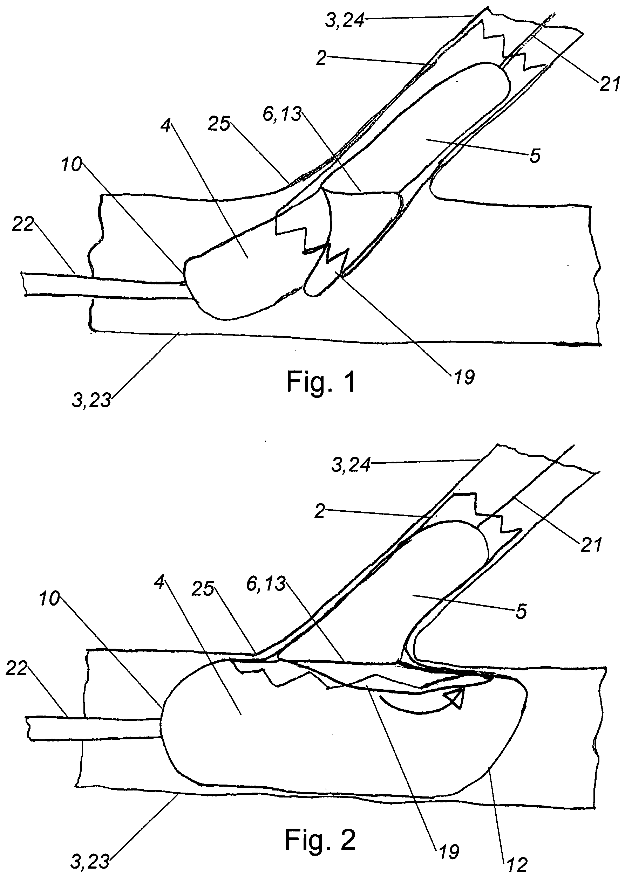

Die Fig. 1 bis 11 zeigen bevorzugte Ausführungsformen einer Vorrichtung zur vorgebbaren Anordnung eines Stents 2 in einem Blutgefäß 3, wobei die Vorrichtung einen ersten aufblasbaren Hohlkörper 4 und einen zweiten aufblasbaren Hohlkörper 5 aufweist, wobei - in einem schlaffen Zustand der beiden aufblasbaren Hohlkörper 4,5 - der erste Hohlkörper 4 und der zweite Hohlkörper 5 jeweils im Wesentlichen die Form eines Zylinders aufweisen. Der schlaffe Zustand der beiden aufblasbaren Hohlkörper 4,5 wird zum Zwecke der besseren Lesbarkeit in Folge lediglich als der schlaffe Zustand bezeichnet. Aufblasbar bedeutet in diesem Sinne, dass die beiden Hohlkörper 4,5 ähnlich einem Ballon Ihre Form verändern, wenn die beiden Hohlkörper 4,5 mit einem Inflationsfluid gefüllt werden. Im Wesentlichen die Form eines Zylinders aufweisend wird in diesem Zusammenhang sehr weitläufig gesehen. Ein im Wesentlichen die Form eines Zylinders aufweisender Körper hat eine längliche Form, wobei der Querschnitt durchaus komplexere Formen annehmen kann. Auch kann der Querschnitt über die Längsachse variieren, und beispielsweise zu den Enden hin kontinuierlich abnehmen.

Eine Längserstreckung des ersten Hohlkörpers 4 ist, im schlaffen Zustand, im Wesentlichen fluchtend zu einer Längserstreckung des zweiten Hohlkörpers 5 angeordnet. Im Wesentlichen fluchtend bedeutet in diesem Sinne, dass die Längserstreckung des ersten Hohlkörpers 4 und die Längserstreckung des zweiten Hohlkörpers 5 einen Winkel von bis zu 15° einschließen können. Dadurch kann die Vorrichtung gut zu der gewünschten Stelle transportiert werden, wobei die Vorrichtung bei der Bifurkation durch eine gegebenenfalls vorhandene leichte Krümmung leichter in den Nebenast 24 eingeführt und richtig positioniert werden kann.

In einem vorgebbar aufgeblasenen Zustand der beiden Hohlkörper 4,5 weisen der erste Hohlkörper 4 und der zweite Hohlkörper 5 jeweils im Wesentlichen die Form eines Zylinders auf, wobei die Längserstreckung des zweiten Hohlkörpers 5 einen vorgebbaren Winkel zwischen 20° und 160° zur Längserstreckung des ersten Hohlkörpers 4 einnimmt. Der vorgebbar aufgeblasene Zustand der beiden Hohlkörper 4,5 wird zum Zwecke der besseren Lesbarkeit in Folge als der aufgeblasene Zustand bezeichnet. Der vorgebbare Winkel im aufgeblasenen Zustand wird zwischen dem distalen Ende des ersten Hohlkörpers 4 und dem zweiten Hohlkörper 5 gemessen. Das distale Ende des aufgeblasenen ersten Hohlkörpers 4 ist dabei jenes Ende des ersten Hohlkörpers 4, welches im vorgesehenen Einsatz im Blutgefäß 3 dem arteriellen Zugang abgewandt ist.

Dadurch ergibt sich der Vorteil, dass die Einengung an der Bifurkation vollständig beseitigt werden kann. Dabei kann der Stent 2 des Nebenastes 24 vollständig an die Gefäßwand des Hauptastes 23 im Bereich des Ostiums 25 angelegt werden, wodurch ein stabiler Zusammenhalt der Gefäßwand des Hauptastes 23 mit dem Stent 2 des Nebenastes 24 erreicht werden kann. Weiters wird dadurch verhindert, dass Teile des Stents 2 des Nebenastes 24 in den Hauptast 23 hineinragen und zu Komplikationen führen. Das vollständige Anlegen des Stents 2 des Nebenastes 24 an das Ostium 25 kann mit einem Schritt erfolgen, wodurch die Zeit des medizinischen Eingriffes kurz gehalten werden kann, wodurch die Sicherheit des Patienten erhöht werden kann.

Der erste Hohlkörper 4 und der zweite Hohlkörper 5 können insbesondere aus einem für Ballonkatheter geeignetem Material ausgebildet sein. Solche Materialien können beispielsweise thermoplastische Polymere, Polyethylene, Co-Polymere oder Nylon sein, um nur einige zu nennen.

Besonders bevorzugt kann vorgesehen sein, dass der erste Hohlkörper 4 und/oder der zweite Hohlkörper 5 aus einem non-compliant Material ausgebildet sind. Insbesondere kann vorgesehen sein, dass eine Wand 12 des ersten Hohlkörpers 4 und/oder eine Wand des zweiten Hohlkörpers 5 aus einem non-compliant Material ausgebildet sind. Ein Ballonkatheter aus einem non-compliant Material, auch oft als non-compliant Ballonkatheter bezeichnet, dehnt sich zunächst bis zu einer vorgegebenen Größe aus. Nach Erreichen der vorgegebenen Größe verändert sich der Durchmesser mit zunehmenden Druck nur geringfügig. Dadurch kann die Gefahr einer Überdehnung des Blutgefäßes 3 gering gehalten werden.

Besonders bevorzugt kann vorgesehen sein, dass der zweite Hohlkörper 5 im Wesentlichen unmittelbar angrenzend an den ersten Hohlkörper 4 angeordnet ist. Dadurch kann eine besonders kompakte Form der Vorrichtung erzielt werden, welche leicht einzuführen und zu bedienen ist. Weiters kann dadurch eine gute Kraftwirkung auf den Stent 2 des Nebenastes 24 im Bereich des Ostiums 25 erzielt werden.

Weiters kann bevorzugt vorgesehen sein, dass - im schlaffen Zustand - eine Stirnseite 6 des zweiten Hohlkörpers 5 an einer Stirnseite des ersten Hohlkörpers 4 angrenzt. Hierbei ist zu beachten, dass der erste Hohlkörper 4 beim Aufblasen seine Form verändern kann, und daher die Stirnseite des ersten Hohlkörpers 4 im schlaffen Zustand nicht zwangsweise die Stirnseite des ersten Hohlkörpers 4 im aufgeblasenen Zustand sein muss. Durch diese Form und/oder Anordnung im schlaffen Zustand kann die Vorrichtung besonders gut im schlaffen Zustand durch die Blutgefäße 3 bewegt werden.

Besonders bevorzugt kann vorgesehen sein, dass an der Vorrichtung eine Markierung angeordnet ist, insbesondere im Bereich der dem ersten Hohlkörper 4 zugewandten Stirnseite des zweiten Hohlkörpers 5, und welche Markierung insbesondere röntgensichtbar ist. Röntgensichtbar bedeutet in diesem Zusammenhang, dass die Markierung also Röntgenbild einen guten Kontrast zum umgebenden Bereich aufweist. Dadurch kann die behandelnde Person die Vorrichtung vor dem Aufblasen gezielt positionieren.

Insbesondere kann vorgesehen sein, dass - im aufgeblasenen Zustand - der zweite Hohlkörper 5 an einer Mantelfläche des ersten Hohlkörpers 4 angeordnet ist. Dadurch steht der zweite Hohlkörper 5 im aufgeblasenen Zustand von dem ersten Hohlkörper 4 ab, wodurch die Vorrichtung im aufgeblasenen Zustand sich der Form der Bifurkation anpassen kann, wodurch auch der Stent 2 gut an dem Blutgefäß 3 im Bereich der Bifurkation befestigt werden kann.

Besonders bevorzugt kann weiters vorgesehen sein, dass - im aufgeblasenen Zustand - der zweite Hohlkörper 5 beidseitig in Längserstreckung des ersten Hohlkörpers 4 von der Mantelfläche des ersten Hohlkörpers 4 umgeben ist. Hierbei sieht die Vorrichtung im aufgeblasenen Zustand im Querschnitt wie ein schiefes T aus, wobei in einer Bifurkation der zweite Hohlkörper 5 im Nebenast 24 sitzt, und der erste Hohlkörper 4 sich im Bereich des Ostiums 25 im Hauptast 23 befindet, und den Stent 2 des Nebenastes 24 im Bereich des Hauptastes 23 an die Gefäßwand des Blutgefäßes 3 drückt. Hierbei erstreckt sich, von dem arteriellen Zugang des Katheters aus gesehen, der erste Hohlkörper 4 sowohl distal als auch proximal vom Ostium 25, wodurch der Stent 2 besonders gut an die Form der Bifurkation angepasst werden können.

Insbesondere kann vorgesehen sein, dass - im aufgeblasenen Zustand - der zweite Hohlkörper 5 im Wesentlichen in der Mitte der Längserstreckung der Mantelfläche des ersten Hohlkörpers 4 angeordnet ist.

Bei dem vorgesehenen Einsatz der Vorrichtung in einer Bifurkation kann insbesondere vorgesehen sein, dass im schlaffen Zustand der zweite Hohlkörper 5 im Nebenast 24 angeordnet ist, und der erste Hohlkörper im Wesentlichen fluchtend zum zweiten Hohlkörper 5 im proximalen Teil des Hauptastes 23 angeordnet ist, und dass die Form des ersten Hohlkörpers beim Aufblasen derart veränderbar ist, dass sich der erste Hohlkörper beim Aufblasen distal wesentlich stärker ausbreitet als proximal.

Bevorzugt kann vorgesehen sein, dass ein Durchmesser des ersten Hohlkörpers 4 im aufgeblasenen Zustand größer ist, als ein Durchmesser des zweiten Hohlkörpers 5 im aufgeblasenen Zustand. Da beide Hohlkörper 4,5 lediglich im Wesentlichen die Form eines Zylinders aufweisen und daher recht komplexe Formen annehmen können, ist in diesem Sinne der Durchmesser eines der beiden Hohlkörper 4, 5 der Durchmesser des Umkreises des Querschnitts normal zur Längsachse, welcher in der Mitte der Längsachse angeordnet ist, wobei der gegenständliche Hohlkörper 4,5 mit einem Überdruck von 5 bar außerhalb eines Körpers aufgeblasen ist. Dadurch entspricht die aufgeblasene Vorrichtung mehr der Form der Bifurkation.

Insbesondere kann vorgesehen sein, dass der Durchmesser der beiden Hohlkörper 4, 5 zwischen 1 mm und 10 mm, insbesondere zwischen 2 mm und 5 mm beträgt.

Besonders bevorzugt kann vorgesehen sein, dass der Durchmesser des zweiten Hohlkörpers 5 im aufgeblasenen Zustand mit zunehmendem Abstand vom ersten Hohlkörper 4 stetig abnimmt, daher, dass der zweite Hohlkörper 5 im Wesentlichen eine konische Form aufweist. Dadurch kann die Vorrichtung leichter in den Nebenast 24 eingeführt werden.

Bevorzugt kann weiters vorgesehen sein, dass der erste Hohlkörper 4 und/oder der zweite Hohlkörper 5 für einen Überdruck von 10 bar, vorzugsweise 20 bar, insbesondere 30 bar, ausgebildet ist. Dadurch kann eine Verengung des Blutgefäßes 3 gut aufgeweitet, und der Stent 2 zuverlässig fixiert werden.

Besonders bevorzugt kann vorgesehen sein, dass an einem proximalen Ende 10 des ersten Hohlkörpers 4 im aufgeblasenen Zustand eine Zuleitung 22 angeordnet ist, über welche das Inflationsfluid in die beiden Hohlkörper 4,5 einleitbar ist. Das proximale Ende des aufgeblasenen ersten Hohlkörpers 4 ist jenes Ende, welches dem arteriellen Zugang zugewandte ist. Das Inflationsfluid kann beispielsweise eine NaCl Lösung mit einem Kontrastmittel sein.

Hierbei kann vorgesehen sein, dass der Hohlraum des ersten Hohlkörpers 4 mit dem Hohlraum des zweiten Hohlkörpers 5 verbunden ist, so dass beim Aufblasen sowohl im ersten Hohlkörper 4 als auch im zweiten Hohlkörper 5 der gleiche Druck vorherrscht.

Insbesondere kann vorgesehen sein, dass der zweite Hohlkörper 5 derart ausgebildet ist, dass sich der zweite Hohlkörper 5 vor dem ersten Hohlkörper 4 ausdehnt. Dies kann vorzugsweise dadurch erfolgen, dass der erste Hohlkörper 4 und der zweite Hohlkörper 5 unterschiedlich dehnbar sind. Dadurch kann die Vorrichtung zuverlässig im Nebenast 24 verankert werden, bevor der Stent 2 durch den ersten Hohlkörper 4 verformt wird.

Alternativ kann vorgesehen sein, dass der erste Hohlkörper 4 und der zweite Hohlkörper 5 getrennt voneinander aufblasbar sind. Dies kann beispielsweise dadurch erfolgen, dass an der Zuleitung 22 eine Nebenleitung angeordnet ist, welche dazu ausgebildet ist, lediglich den zweiten Hohlkörper 5 aufzublasen.

Dadurch kann beispielsweise bei der vorgesehenen Verwendung zuerst der zweite Hohlkörper 5 im Nebenast 24 aufgeblasen werden, um die Vorrichtung zu fixieren, und erst in einem zweiten Schritt der erste Hohlkörper 4 aufgeblasen werden, um den Stent 2 an die Bifurkation anzupassen.

In der Regel werden Führungsdrähte 21 verwendet, um Ballonkatheter zur vorgesehenen Stelle zu transportieren und/oder zu leiten. Besonders bevorzugt kann daher vorgesehen sein, dass durch den ersten Hohlkörper 4 und den zweiten Hohlkörper 5 ein Führungsschlauch 20 zur Führung eines Führungsdrahtes 21 angeordnet ist. Hierbei kann der Führungsdraht 21 im Führungsschlauch 20, welcher auch als Drahtkanal bezeichnet werden kann, bewegbar angeordnet werden. Bei der vorgesehenen Verwendung wird der Führungsdraht 21 in dem Nebenast 24 angeordnet und in weiterer Folge der zweite Hohlkörper 5 entlang des Führungsdrahtes 21 ebenfalls im Nebenast 24 angeordnet. Dadurch kann die Vorrichtung für die vorgesehene Verwendung gut in der Bifurkation angeordnet werden. Dadurch, dass der Führungsschlauch 20 innerhalb der beiden Hohlkörper 4, 5 angeordnet ist, kann die Vorrichtung besonders zuverlässig entlang des Führungsdrahtes 21 bewegt werden.

Gemäß einer weiteren, nicht dargestellten Ausführungsform der gegenständlichen Erfindung kann vorgesehen sein, dass wenigstens ein weiterer Führungsschlauch bzw. Drahtkanal vorgesehen ist, bzw. an dem Hohlkörper bzw. an dem ersten Führungsschlauch 20 befestigt ist. Dadurch kann ein zweiter Führungsdraht verwendet werden, um den Hohlkörper einfacher auszurichten. Dadurch kann besonders einfach mittels eines weiteren Drahtes der erste Hohlkörper 4 derart entlang seiner Längserstreckung gedreht werden, dass der zweite Hohlkörper 5 sich beim Aufblasen in die angestrebte Richtung erstreckt.

In Fig. 1 bis Fig. 11 sind die Proportionen der Vorrichtung zur besseren Darstellung der Merkmale verzehrt dargestellt. Insbesondere bezüglich dem schlaffen Zustand ist da Verhältnis Länge der Vorrichtung zu Durchmesser der Vorrichtung wesentlich kleiner dargestellt als in der Realität zu erwarten.

Fig. 1 zeigt die erste bevorzugte Ausführungsform der Vorrichtung im schlaffen Zustand in einer vorgesehenen Position innerhalb einer Bifurkation eines Blutgefäßes 3. Die Bifurkation in Fig. 1 besteht aus einem geraden Hauptast 23, von welchem in einem Winkel von ca. 45° ein Nebenast 24 abzweigt. Im Nebenast 24 ist bereits der Stent 2 angeordnet. Zum Zwecke der Übersichtlichkeit wird der Stent 2 in Fig. 1 und Fig. 2 nur als Umriss mit gezackten Enden dargestellt. Ein Führungsdraht 21 führt von dem Hauptast 23 durch das Ostium 25 in den Nebenast 24, wobei am Führungsdraht 21 die erste bevorzugte Ausführungsform der Vorrichtung bewegbar angeordnet ist.

In Fig. 1 ist der zweite Hohlkörper 5 innerhalb des Stents 2 des Nebenastes 24 angeordnet, wobei die Stirnseite 6 des zweiten Hohlkörpers 5, welche dem ersten Hohlkörper 4 zugewandt ist, sich auf der Höhe des Ostiums 25 befindet und mit einem Winkel von 45° schräg zur Längsachse des zweiten Hohlkörpers 5 orientiert ist. Der erste Hohlkörper 4 ist unmittelbar angrenzend an den zweiten Hohlkörper 5 angeordnet, wobei sich der erste Hohlkörper 4 bereichsweise innerhalb des Stents 2 des Nebenastes 24 befindet.

Fig. 2 zeigt die erste bevorzugte Ausführungsform der Vorrichtung im aufgeblasenen Zustand in einer vorgesehenen Position innerhalb des Blutgefäßes 3 aus Fig. 1. Der aufgeblasene zweite Hohlkörper 5 liegt innen an dem Stent 2 des Nebenastes 24 an, womit die Position der Vorrichtung gegenüber der Bifurkation verankert ist. Der aufgeblasene erste Hohlkörper 4 weist nun im Wesentlichen die Form eines Zylinders auf, wobei die Längsachse des aufgeblasenen ersten Hohlkörpers 4 mit der Längsachse des Hauptastes 23 fluchtet.

Gemäß Fig. 2 schließen die Längsachse des aufgeblasenen zweiten Hohlkörpers 5 und die Längsachse des aufgeblasenen ersten Hohlkörpers 4 einen Winkel von 45° ein. Weiters grenzt der zweite Hohlkörper 5 an den mittleren Bereich des ersten Hohlkörpers 4 an.

Der erste Hohlkörper 4 hat sich beim Aufblasen distal viel stärker ausgebreitet als proximal. Jene Bereiche des Stents 2 des Nebenastes 24, in welchen der erste Hohlkörper 4 im schlaffen Zustand angeordnet war, sind im aufgeblasenen Zustand durch den ersten Hohlkörper 4 an die Innenseite der Gefäßwand des Hauptastes 23 gedrückt, wie durch den Pfeil in Fig. 2 angedeutet, wodurch die Form des Stents 2 an die Form der Bifurkation des Blutgefäßes 3 angepasst ist, und kein Teil des Stents 2 in das Blutgefäß 3 ragt.

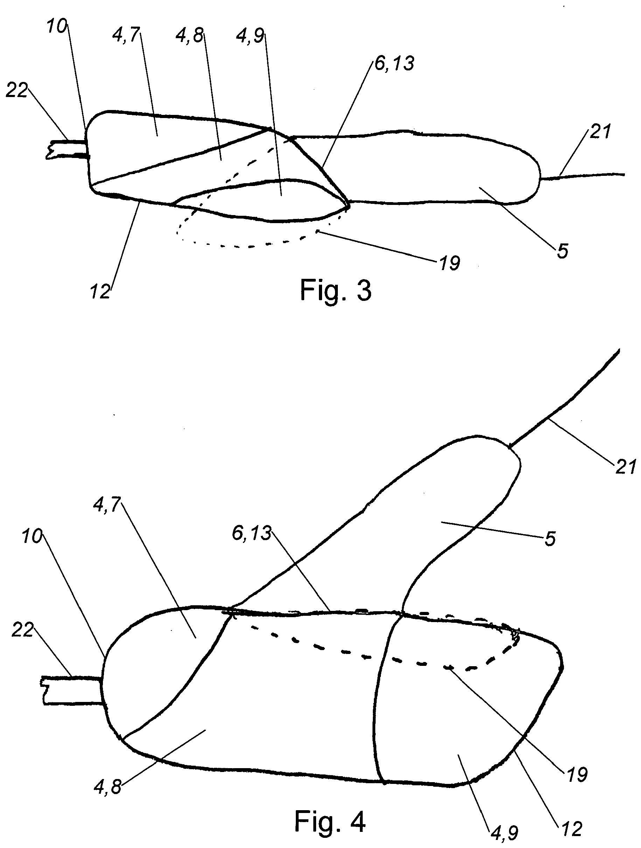

Um den ersten Hohlkörper 4 inhomogen aufzublasen, daher, dass der erste Hohlkörper 4 im schlaffem Zustand eine andere Form und Orientierung aufweist als im aufgeblasenem Zustand, kann wie in Fig. 3 und Fig. 4 dargestellt, bevorzugt vorgesehen sein, dass die Wand 12 des ersten Hohlkörpers 4 aus wenigstens einem ersten Bereich 7 und einem zweiten Bereich 8 besteht, und dass der erste Bereich 7 und der zweite Bereich 8 unterschiedlich dehnbar sind. Unterschiedlich dehnbar bedeutet in diesem Zusammenhang, dass sich der erste Bereich 7 und der zweite Bereich 8 bei sich verändernden Druck im ersten Hohlkörper 4 unterschiedlich stark ausdehnen, daher deren Fläche verändern. Dadurch kann der erste Hohlkörper 4 beim Aufblasen seine Form verändern.

Diese unterschiedliche Dehnbarkeit kann beispielsweise dadurch erreicht werden, dass der erste Bereich 7 und der zweite Bereich 8 unterschiedliche Wanddicken aufweisen. Hierbei kann beispielsweise vorgesehen sein, dass der erste Bereich 7 eine erste Wanddicke aufweist, und der zweite Bereich 8 eine zweite Wanddicke aufweist, wobei die erste Wanddicke größer ist als die zweite Wanddicke. Dadurch dehnt sich der zweite Bereich 8 stärker mit zunehmendem Druck aus als der erste Bereich 7, wodurch der erste Hohlkörper 4 beim Aufblasen seine Form verändert.

Alternativ kann diese unterschiedliche Dehnbarkeit dadurch erreicht werden, dass der erste Bereich 7 und der zweite Bereich 8 unterschiedlich elastisch sind. Hierbei bezieht sich die unterschiedliche Elastizität auf unterschiedliche E-Module der verwendeten Materialien. Beispielsweise kann hierfür der erste Hohlkörper 4 aus unterschiedlichen Materialien gefertigt sein. Alternativ kann aber auch vorgesehen sein, dass der erste Hohlkörper 4 aus einem Material gefertigt ist, dessen mechanische Eigenschaften in einem weiteren Fertigungsschritt bereichsweise gezielt verändert werden, beispielsweise durch eine Behandlung mit Chemikalien, durch äußere Bestrahlung, oder Einprägen einer Oberflächestruktur. Jene Materialen, aus denen der erste Hohlkörper 4 bestehen kann, können mitunter sehr komplexe Dehnungskurven aufweisen. Hierbei kann unter unterschiedlich elastisch auch verstanden werden, dass sich ein Bereich beim Aufblasen am Anfang stärker ausdehnt als ein anderer Bereich, im Endeffekt aber beide Bereiche 7,8 gleich stark ausgedehnt haben.

Weiters kann vorgesehen sein, dass die Übergänge der unterschiedlich dehnbaren Bereiche 7,8 fließend sind, und nicht abrupt.

Bezüglich der Anordnung der Bereiche 7,8 kann besonders bevorzugt vorgesehen sein, dass im aufgeblasenen Zustand der erste Bereich 7 zwischen dem proximalen Ende 10 des ersten Hohlkörpers 4 und dem zweiten Hohlkörper 5 angeordnet ist, und dass der zweite Bereich 8 dehnbarer ist als der erste Bereich 7. Dadurch dehnt sich der proximale Bereich des ersten Hohlkörpers 4 weniger stark aus als der restliche Bereich.

Gemäß Fig. 3 und Fig. 4 kann vorgesehen sein, dass im aufgeblasenen Zustand am distalen Ende des ersten Hohlkörpers 4 ein dritter Bereich 9 angeordnet ist, welcher dritter Bereich 9 dehnbarer ist als der erste Bereich 7 oder der zweite Bereich 8. Dadurch kann sich der erste Hohlkörper 4 beim Aufblasen in die distale Richtung stark ausweiten, wodurch der erste Hohlkörper 4 gut von der ursprünglichen Form im schlaffen Zustand in den Hauptast 23 ausdehnen kann.

Um zu verhindern, dass der Stent 2 des Nebenastes 24 beim Aufblasen den ersten Hohlkörper 4 beschädigt, kann vorgesehen sein, dass in einem dem ersten Hohlkörper 4 zugewandten Bereich des zweiten Hohlkörpers 5 ein Schutzlappen 19 angeordnet ist. Dieser Schutzlappen 19 kann insbesondere aus einem widerstandsfähigeren Material als der erste Hohlkörper 4 ausgebildet sein.

Gemäß der ersten bevorzugten Ausführungsform in Fig. 3 und Fig. 4 kann vorgesehen sein, dass der Schutzlappen 19 schürzenartig um die Stirnseite 6 des zweiten Hohlkörpers 5, welche an den ersten Hohlkörper 4 angrenzt, befestigt ist, und an jener Seite des ersten Hohlkörpers 4 angeordnet ist, welche sich beim Aufblasen in Richtung des zweiten Hohlkörpers 5 bewegt. Dadurch kann jene Stelle des ersten Hohlkörpers 4 effektiv geschützt werden, welche den Stent 2 des Nebenastes 24 am meisten verbiegt.

Dass die Vorrichtung, und insbesondere der erste Hohlkörper 4, beim Aufblasen vorgebbar die Form verändert, kann auch auf alternative Weise erreicht werden. Diese alternativen Ausführungsformen können dabei alleine das gewünschte Ziel erreichen, aber auch eine Kombination dieser diversen Ausführungsformen ist möglich.

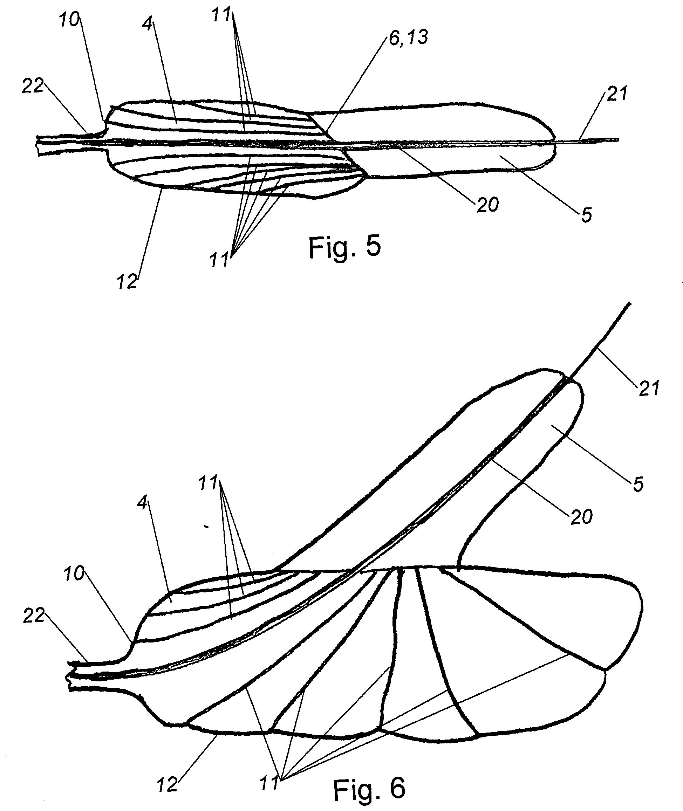

Gemäß der zweiten bevorzugten Ausführungsform, welche in Fig. 5 und Fig. 6 dargestellt ist, kann vorgesehen sein, dass im Inneren des ersten Hohlkörpers 4 eine vorgebbare Mehrzahl an Fäden 11, welche mit der Wand 12 des ersten Hohlkörpers 4 verbunden sind, angeordnet sind, und dass die Fäden 11 im aufgeblasenen Zustand zur Vorgabe der Form des ersten Hohlkörpers 4 gespannt sind. Hierbei wird die Länge, und/oder Dicke und/oder Elastizität der einzelnen Fäden 11 derart vorgegeben, dass sich der erste Hohlkörper 4 bei der vorgesehenen Verwendung sich distal stärker ausbreiten kann als proximal. Dadurch kann erreicht werden, dass der erste Hohlkörper 4 beim Aufblasen seine Form vorgebbar verändert, da durch die Anordnung der im aufgeblasenen Zustand zugbelasteten Fäden 11 die Form des ersten Hohlkörpers 4 vorgegeben werden kann. Hierbei wird eine weitere Ausdehnung des ersten Hohlkörpers 4 in die Längsrichtungen der Fäden durch die Länge, und/oder Dicke und/oder Elastizität der einzelnen Fäden 11 vorgebbar begrenzt. In Fig. 5 und Fig. 6 sind der erste Hohlkörper 4 und der zweite Hohlkörper 5 durchsichtig dargestellt, weshalb die Fäden 11 im Inneren des ersten Hohlkörpers 4 als durchgezogene Linie dargestellt sind.

Insbesondere kann vorgesehen sein, dass die Fäden 11 aus einem Polymer ausgebildet sind.

Gemäß der zweiten bevorzugten Ausführungsform kann vorgesehen sein, dass eine vorgebbare Anzahl der Fäden 11 jeweils mit einem Ende an einem, an den zweiten Hohlkörper 5 angrenzenden Teil 13 der Wand 12 des ersten Hohlkörpers 4 befestigt sind. Dadurch lässt sich der erste Hohlkörper 4 im aufgeblasenen Zustand gut in seiner Form vorgeben.

Insbesondere kann dabei vorgesehen sein, dass die Fäden lediglich an den Enden an der Wand 12 des ersten Hohlkörpers 4 befestigt sind, also bis auf die Enden frei im ersten Hohlkörper 4 angeordnet sind.

Hierbei kann insbesondere vorgesehen sein, dass jene Fäden 11, welche mit dem anderen Ende am distalen Ende des ersten Hohlkörpers 4 befestigt sind im aufgeblasenen Zustand länger sind als jene Fäden 11, welche mit dem anderen Ende am proximalen Ende 10 des ersten Hohlkörpers 4 befestigt sind. Dadurch kann sich der erste Hohlkörper 4 beim Aufblasen distal viel mehr ausbreiten als proximal.

Alternativ kann vorgesehen sein, dass die Fäden 11 nach einem komplexen dreidimensionalen Muster, beispielsweise spinnennetzartig, angeordnet sind.

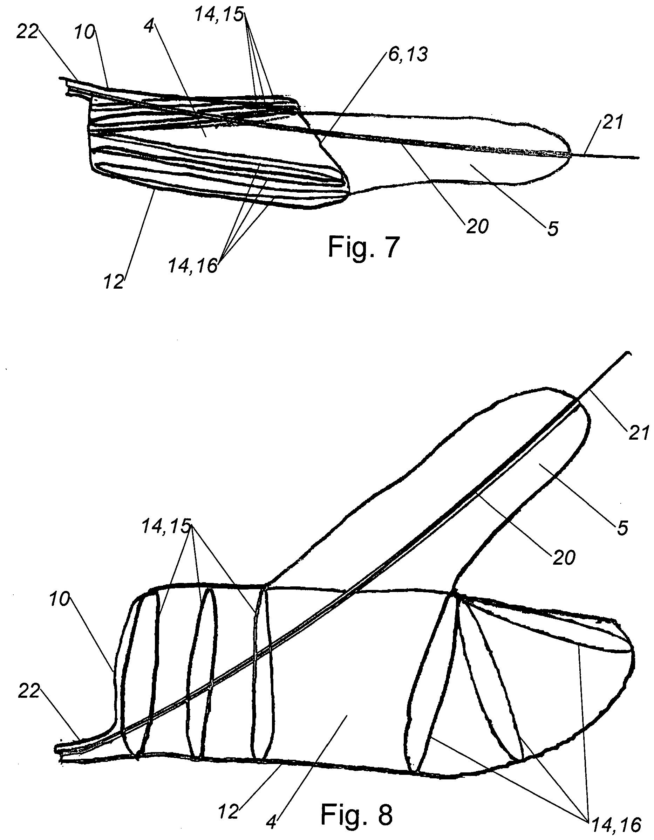

Gemäß einer dritten Ausführungsform, welche in Fig. 7 und Fig. 8 dargestellt ist, kann vorgesehen sein, dass an der Wand 12 des ersten Hohlkörpers 4 Stützelemente 14 angeordnet sind, und dass die Stützelemente 14 im aufgeblasenen Zustand die Form des ersten Hohlkörpers 4 vorgeben. Dadurch kann erreicht werden, dass der erste Hohlkörper 4 beim Aufblasen seine Form vorgebbar verändert.

Die Stützelemente 14 können hierbei an der Außenseite der Wand 12 befestigt sein, oder an der Innenseite der Wand 12. Weiters wird in Fig. 7 und Fig. 8 wird der erste Hohlkörper 4 und der zweite Hohlkörper 5 durchsichtig dargestellt.

Insbesondere kann vorgesehen sein, dass die Stützelemente 14 im schlaffen Zustand im Wesentlichen elliptisch und im aufgeblasenen Zustand im Wesentlichen kreisförmig sind. Dadurch kann der erste Hohlkörper 4 im schlaffem Zustand schmaler sein als im aufgeblasenen Zustand.

Im schlaffen Zustand kann insbesondere die Hauptachse der im Wesentlichen elliptisch-förmigen Stützelemente 14 im Wesentlichen parallel zur Längsachse des zweiten Hohlkörpers 5 sein, während im aufgeblasenen Zustand die im Wesentlichen kreisförmigen Stützelemente 14 normal zur Längsachse des ersten Hohlkörpers 4 angeordnet sind.

Insbesondere kann vorgesehen sein dass die Stützelemente 14 flexibel ausgebildet sind, sodass die Stützelemente 14 deren Form verändern können. Weiters kann vorgesehen sein, dass ein Stützelement 14 im schlaffen Zustand aus zwei flexiblen, länglichen, und im Wesentlichen parallelen Elementen bestehen, welche an ihren Endpunkten mit Scharnieren verbunden sind.

Dabei können bevorzugt die Stützelemente 14 einstückig ausgebildet sein, wobei das Scharnier als eine Stelle mit einem besonders dünnen Querschnitt ausgebildet ist.

Besonders bevorzugt kann vorgesehen sein, dass die Stützelemente 14 einer ersten Gruppe 15 oder einer zweiten Gruppe 16 zugeteilt sind, und dass im aufgeblasenen Zustand der an dem zweiten Hohlkörper 5 angrenzender Teil 13 der Wand 12 des ersten Hohlkörpers 4 zwischen der ersten Gruppe 15 und der zweiten Gruppe 16 angeordnet ist, wodurch sich der erste Hohlkörper 4 im aufgeblasenen Zustand sowohl proximal als auch distal vom zweiten Hohlkörper 5 erstrecken kann.

Im schlaffen Zustand der dritten bevorzugten Ausführungsform, welche in Fig. 7 dargestellt ist, sind die Stützelemente 14 länglich und nah beieinander angeordnet, wobei die Stützelemente 14 in zwei Gruppen 15,16 unterteilt sind. Der Führungsschlauch 20 führt hierbei durch die erste Gruppe 15 von Stützelementen.

Fig. 8 zeigt die dritte bevorzugte Ausführungsform im aufgeblasenen Zustand, wobei die Stützelemente 14 im Wesentlichen kreisförmig geformt sind. Da eine Kreis seitlich projiziert einen Strich ergibt, wurden die kreisförmigen Stützelemente 14 in Fig. 8 zum intuitiveren Verständnis des Betrachters als Ellipsen dargestellt. Die Stützelemente 14 der ersten Gruppe 15 sind gemäß Fig. 8 in einem gleichbleibenden Abstand zueinander angeordnet, während die Stützelemente 14 der zweiten Gruppe 16 nach dem Aufblasen fächerartig ausgebreitet angeordnet sind, wodurch die Form des ersten Hohlkörpers 4 im aufgeblasenen Zustand vorgegeben ist, da ein weiteres Verformen des ersten Hohlkörpers 4 durch die Stützelemente 4 begrenz ist.

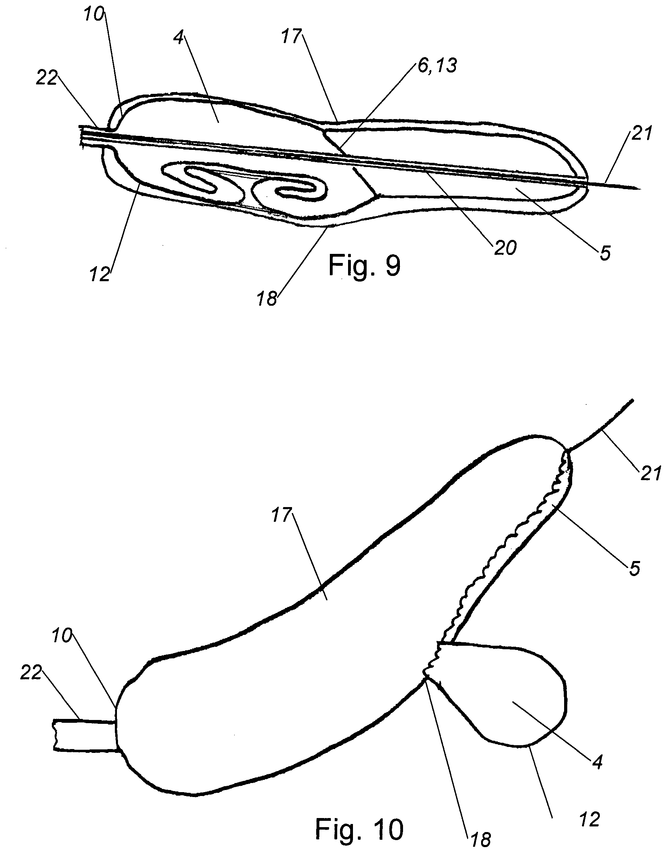

Gemäß der vierten bevorzugten Ausführungsform der Vorrichtung, welche in Fig. 9 bis Fig. 11 dargestellt ist, kann vorgesehen sein, dass der erste Hohlkörper 4 im schlaffen Zustand in einer im Wesentlichen zylinderförmigen Transporthülle 17 angeordnet ist. Dadurch wird der erste Hohlkörper 4 im schlaffen Zustand in eine von der Transporthülle 17 vorgegebene Form gebracht. Der erste Hohlkörper 4 befindet ist hierbei in der Transporthülle 17 zusammengefaltet, wobei die Form des ersten Hohlkörpers 4 im schlaffen aber nicht-zusammengefalteten Zustand bereits im Wesentlichen der Form des aufgeblasenen ersten Hohlkörpers 4 entsprechen kann. Dadurch kann der erste Hohlkörper 4 in einer vorgegebenen Form zur Bifurkation im Blutgefäß 3 transportiert werden und durch Entfernen der Transporthülle 17 in die vorgegebene Form des aufgeblasenen Zustandes gebracht werden.

Insbesondere kann vorgesehen sein, dass der erste Hohlkörper 4 und der zweite Hohlkörper 5 im schlaffen Zustand in der Transporthülle 17 angeordnet sind, wodurch sich die Vorrichtung besser durch das Blutgefäß 3 bewegen lässt.

Die Transporthülle 17 kann besonders bevorzugt dadurch entfernbar ausgebildet werden, indem die Transporthülle 17 in Richtung der Zuleitung 22 wegziehbar angeordnet ist.

Um die Transporthülle 17 von dem ersten Hohlkörper 4, und insbesondere dem zweiten Hohlkörper 5, wegzuziehen, insbesondere um die Transporthülle 17 separat von dem ersten Hohlkörper 4 und dem zweiten Hohlkörper 5 aus dem Blutgefäß 3 oder dem Körper des Patienten zu entfernen kann bevorzugt vorgesehen sein, dass die Transporthülle 17 an einem längliches Zugelement, insbesondere einem Faden und/oder einem weiteren Draht, befestigbar ausgebildet ist. Dadurch kann die Transporthülle 17, welche die Form des ersten Hohlkörpers 4 bis zum Transport zur Bifurkation vorgibt, bei der Bifurkation entfernt werden, wodurch der Hohlkörper 4 bei der Bifurkation ungehindert in die vorgegebene Form des aufgeblasenen Zustandes gebracht werden kann. Durch das Zugelement kann dann die Transporthülle 17 aus dem Blutgefäß 3 oder dem Körper des Patienten entfernt werden, wodurch etwaige Komplikationen mit der Transporthülle 17 weitgehend verhindert werden können.

Insbesondere kann die Transporthülle 17 gegenüber dem ersten Hohlkörper 4, und insbesondere dem zweiten Hohlkörper 5, lose angeordnet sein, also nicht befestigt sein. Dadurch kann die Transporthülle 17 von dem ersten Hohlkörper 4, und insbesondere dem zweiten Hohlkörper 5, besonders einfach entfernt werden.

Besonders bevorzugt kann vorgesehen sein, dass die Transporthülle 17 für einen maximalen Druck von 10 bar, insbesondere 5 bar, ausgebildet ist. Dadurch platzt die Transporthülle beim Aufblasen der Vorrichtung, wodurch die Transporthülle 17 auf eine besonders einfache Art und Weise beim Aufblasen entfernt werden kann.

Insbesondere kann vorgesehen sein, dass die Transporthülle 17 Sollbruchstellen 18 aufweist, zum vorgebbaren Aufreisen der Transporthülle 17 beim Aufblasen des ersten Hohlkörpers 4. Dadurch kann die Form des ersten Hohlkörpers 4 nicht nur im schlaffen Zustand und im aufgeblasenen Zustand vorgegeben werden, sondern auch noch die Form des ersten Hohlkörpers 4 während des Aufblasens kontrolliert werden.

Die Sollbruchstellen 18 kann beispielsweise dadurch ausgebildet sein, dass Bereiche der Transporthülle 17 besonders dünn ausgebildet sind, oder durch gezielte Perforationen der Transporthülle 17, wobei viele eng anliegende Perforationen die Transporthülle bei geringerem Druck reißen lassen. Dadurch kann die Sollbruchstelle 18 einen fließenden Verlauf haben, welcher vorgibt, wo und bei welchem Druck die Transporthülle 17 aufreißt.

In Fig. 9 ist zu sehen, wie der erste Hohlkörper 4 und der zweite Hohlkörper 5 im schlaffen Zustand in der Transporthülle angeordnet sind. Hierbei ist der erste Hohlkörper 4 zusammengefaltet. Die Sollbruchstelle 18 der Transporthülle 17 ist hierbei jener Seite zugewandt, welche sich bei Aufblasen stärker ausdehnen soll. Hierbei ist die Sollbruchstelle 18 derart ausgebildet, dass die Transporthülle 17 bei ansteigendem Druck zunächst im Bereich des zweiten Hohlkörpers 5 aufreißt, welcher dem ersten Hohlkörper 4 abgewandt ist, und sich dann der Riss durch die Transporthülle 17 zum proximalen Ende 10 des ersten Hohlkörpers 4 ausbreitet.

Fig. 10 zeigt einen Zwischenschritt beim Aufblasen der vierten bevorzugten Ausführungsform. Hierbei ist die Transporthülle 17 teilweise aufgerissen, wobei die Sollbruchstelle 18 im Bereich des zweiten Hohlkörpers 5 bereits gänzlich aufgerissen ist, aber im Bereich des ersten Hohlkörpers 4 an jener Stelle, welche bei der vorgesehenen Verwendung am Ostium 25 angeordnet ist. Dadurch wird bei der vorgesehenen Verwendung der Stent 2 des Nebenastes 24 zunächst seitlich weggedrückt.

Bevorzugt kann weiters vorgesehen sein, dass die Transporthülle 17 bereichsweise an dem ersten Hohlkörper 4 und/oder an dem zweiten Hohlkörper 5, insbesondere mit wenigstens einer Sollrissstelle, befestigt ist. Beispielsweise kann die Transporthülle 17 an der, der Sollbruchstelle 18 gegenüberliegenden Seite der Vorrichtung befestigt sein. Dadurch kann verhindert werden, dass sich die Transporthülle 17 beim Transport der Vorrichtung zur Bifurkation gegenüber dem ersten Hohlkörper 4 und/oder dem zweiten Hohlkörper 5 verdreht oder verdrillt. Durch die Sollrissstelle, also eine Stelle an welcher die Transporthülle 17 an dem ersten Hohlkörper 4 und/oder an dem zweiten Hohlkörper 5 vorgebbar reißbar befestigt ist, kann die Transporthülle 17 mit einer vorgebbaren Kraft von dem ersten Hohlkörper 4 und/oder an dem zweiten Hohlkörper 5 getrennt werden.

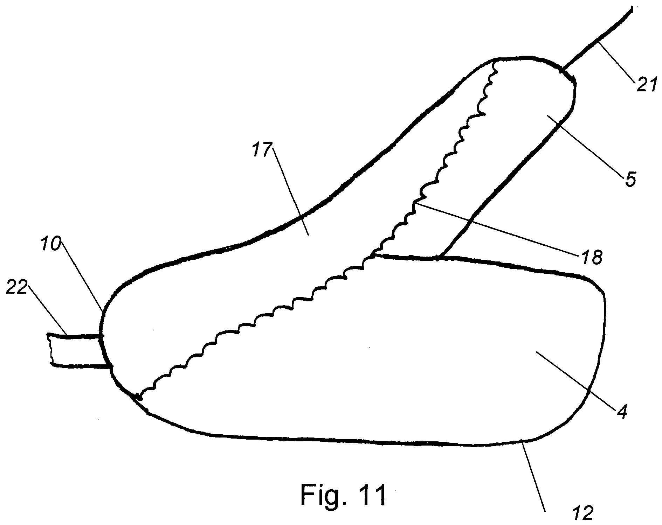

Fig. 11 zeigt die vierte bevorzugte Ausführungsform der Vorrichtung im aufgeblasenen Zustand, wobei die Transporthülle 17 an der Sollbruchstelle 18 gänzlich aufgerissen ist, und der aufgeblasene erste Hohlkörper 4 seine vorgegebene Form hat, wodurch bei der vorgesehenen Verwendung jene Teile des Stents 2 des Nebenastes 24, welche in den Hauptast 23 hineingeragt haben, um das Ostium 25 herum an die Innenseite des Stents 1 des Hauptastes 23 gepresst wurden.

## Patentansprüche

1. Vorrichtung zur vorgebbaren Anordnung eines Stents (2) in einem Blutgefäß (3), wobei die Vorrichtung einen ersten aufblasbaren Hohlkörper (4) und einen zweiten aufblasbaren Hohlkörper (5) aufweist, wobei - in einem schlaffen Zustand der beiden aufblasbaren Hohlkörper (4,5) - der erste Hohlkörper (4) und der zweite Hohlkörper (5) jeweils im Wesentlichen die Form eines Zylinders aufweisen, und wobei eine Längserstreckung des ersten Hohlkörpers (4) im Wesentlichen fluchtend zu einer Längserstreckung des zweiten Hohlkörpers (5) angeordnet ist, wobei - im schlaffen Zustand - eine Stirnseite (6) des zweiten Hohlkörpers (5) an einer Stirnseite des ersten Hohlkörpers (4) angrenzt, **dadurch gekennzeichnet, dass** die Vorrichtung derart ausgebildet ist, dass die Vorrichtung beim Aufblasen vorgebbar die Form verändert, dass - in einem aufgeblasenen Zustand der beiden Hohlkörper (4,5) - der erste Hohlkörper (4) und der zweite Hohlkörper (5) jeweils im Wesentlichen die Form eines Zylinders aufweisen, dass die Längserstreckung des zweiten Hohlkörpers (5) einen vorgebbaren Winkel zwischen 20° und 160° zur Längserstreckung des ersten Hohlkörpers (4) einnimmt, und dass - im aufgeblasenen Zustand - der zweite Hohlkörper (5) an einer Mantelfläche des ersten Hohlkörpers (4) angeordnet ist.

2. Vorrichtung nach Anspruch 1, **dadurch gekennzeichnet, dass** der zweite Hohlkörper (5) im Wesentlichen unmittelbar angrenzend an den ersten Hohlkörper (4) angeordnet ist.

3. Vorrichtung nach Anspruch 1 oder 2, **dadurch gekennzeichnet, dass** - im aufgeblasenen Zustand - der zweite Hohlkörper (5) beidseitig in Längserstreckung des ersten Hohlkörpers (4) von der Mantelfläche des ersten Hohlkörpers (4) umgeben ist.

4. Vorrichtung nach einem der Ansprüche 1 bis 3, **dadurch gekennzeichnet, dass** eine Wand (12) des ersten Hohlkörpers (4) aus wenigstens einem ersten Bereich (7) und einem zweiten Bereich (8) besteht, und dass der erste Bereich (7) und der zweite Bereich (8) unterschiedlich dehnbar sind.

5. Vorrichtung nach Anspruch 4, **dadurch gekennzeichnet, dass** der erste (7) Bereich und der zweite Bereich (8) unterschiedliche Wanddicken aufweisen, oder dass der erste Bereich (7) und der zweite Bereich (8) unterschiedlich elastisch sind.

6. Vorrichtung nach einem der Ansprüche 4 oder 5, **dadurch gekennzeichnet, dass** im aufgeblasenen Zustand der erste Bereich (7) zwischen einem proximalen Ende (10) des ersten Hohlkörpers (4) und dem zweiten Hohlkörper (5) angeordnet ist, und dass der zweite Bereich (8) dehnbarer ist als der erste Bereich (7).

7. Vorrichtung nach einem der Ansprüche 1 bis 6, **dadurch gekennzeichnet, dass** im Inneren des ersten Hohlkörpers (4) eine vorgebbare Mehrzahl an Fäden (11), welche mit einer Wand (12) des ersten Hohlkörpers (4) verbunden sind, angeordnet sind, und dass die Fäden (11) im aufgeblasenen Zustand zur Vorgabe der Form des ersten Hohlkörpers (4) gespannt sind.

8. Vorrichtung nach Anspruch 7, **dadurch gekennzeichnet, dass** eine vorgebbare Anzahl der Fäden (11) jeweils mit einem Ende an einem, an den zweiten Hohlkörper (5) angrenzenden Teil (13) der Wand (12) des ersten Hohlkörpers (4) befestigt sind.

9. Vorrichtung nach einem der Ansprüche 1 bis 8, **dadurch gekennzeichnet, dass** an einer Wand (12) des ersten Hohlkörpers (4) Stützelemente (14) angeordnet sind, und dass die Stützelemente (14) im aufgeblasenen Zustand die Form des ersten Hohlkörpers (4) vorgeben.

10. Vorrichtung nach Anspruch 9, **dadurch gekennzeichnet, dass** die Stützelemente (14) im schlaffen Zustand im Wesentlichen elliptisch und im aufgeblasenen Zustand im Wesentlichen kreisförmig sind.

11. Vorrichtung nach Anspruch 9 oder 10, **dadurch gekennzeichnet, dass** die Stützelemente (14) einer ersten Gruppe (15) oder einer zweiten Gruppe (16) zugeteilt sind, und dass im aufgeblasenen Zustand ein an dem zweiten Hohlkörper (5) angrenzender Teil (13) der Wand (12) des ersten Hohlkörpers (4) zwischen der ersten Gruppe (15) und der zweiten Gruppe (16) angeordnet ist.

12. Vorrichtung nach einem der Ansprüche 1 bis 11, **dadurch gekennzeichnet, dass** der erste Hohlkörper (4) im schlaffen Zustand in einer im Wesentlichen zylinderförmigen Transporthülle (17) angeordnet ist, welche vorzugsweise an dem ersten Hohlkörper (4) und/oder an dem zweiten Hohlkörper (5) befestigt ist.

13. Vorrichtung nach einem der Ansprüche 1 bis 12, **dadurch gekennzeichnet, dass** der erste Hohlkörper (4) und der zweite Hohlkörper (5) getrennt voneinander aufblasbar sind.

## Claims

1. An apparatus for the predeterminable arrangement of a stent (2) in a blood vessel, wherein the apparatus comprises a first inflatable hollow body (4) and a second inflatable hollow body (5), wherein in a limp state of the two inflatable hollow bodies (4, 5), the first hollow body (4) and the second hollow body (5) are each substantially cylindrical, and wherein a longitudinal extension of the first hollow body (4) is arranged substantially in alignment with a longitudinal extension of the second hollow body (5), wherein in the limp state a front face (6) of the second hollow body (5) adjoins a front face of the first hollow body (4), **characterized in that** the apparatus is formed in such a way that the apparatus changes its shape during inflation in a predeterminable manner, that in an inflated state of the two hollow bodies (4, 5) the first hollow body (4) and the second hollow body (5) are each substantially cylindrical, that the longitudinal extension of the second hollow body (5) assumes a predeterminable angle of between 20° and 160° to the longitudinal extension of the first hollow body (4), and that in the inflated state the second hollow body (5) is arranged on a jacket surface of the first hollow body (4).

2. An apparatus according to claim 1, **characterized in that** the second hollow body (5) is arranged substantially directly adjacent to the first hollow body (4).

3. An apparatus according to claim 1 or 2, **characterized in that** in the inflated state the second hollow body (5) is surrounded on both sides in the longitudinal extension of the first hollow body (4) by the jacket surface of the first hollow body (4).

4. An apparatus according to one of the claims 1 to 3, **characterized in that** a wall (12) of the first hollow body (4) consists of at least one first region (7) and a second region (8), and that the first region (7) and the second region (8) are differently extensible.

5. An apparatus according to claim 4, **characterized in that** the first region (7) and the second region (8) have different wall thicknesses, or that the first region (7) and the second region (8) are differently elastic.

6. An apparatus according to one of the claims 4 or 5, **characterized in that** in the inflated state the first region (7) is arranged between a proximal end (10) of the first hollow body (4) and the second hollow body (5), and that the second region (8) is more extensible than the first region (7).

7. An apparatus according to one of the claims 1 to 6, **characterized in that** in the interior of the first hollow body (4) a predetermined plurality of threads (11) are arranged which are connected to a wall (12) of the first hollow body (4), and that in the inflated state the threads (11) are tensioned for predetermining the shape of the first hollow body (4).

8. An apparatus according to claim 7, **characterized in that** a predeterminable number of threads (11) are respectively fastened with one end to a portion (13) of the wall (12) of the first hollow body (4) adjoining the second hollow body (5).

9. An apparatus according to one of the claims 1 to 8, **characterized in that** support elements (14) are arranged on a wall (12) of the first hollow body (4), and that the support elements (14) predetermine the shape of the first hollow body (4) in the inflated state.

10. An apparatus according to claim 9, **characterized in that** the support elements (14) are substantially elliptical in the limp state and substantially circular in the inflated state.

11. An apparatus according to claim 9 or 10, **characterized in that** the support elements (14) are assigned to a first group (15) or a second group (16), and that in the inflated state a portion (13) of the wall (12) of the first hollow body (4) adjoining the second hollow body (5) is arranged between the first group (15) and the second group (16).

12. An apparatus according to one of the claims 1 to 11, **characterized in that** in the limp state the first hollow body (4) is arranged in a substantially cylindrical transport sheath (17) which is preferably fastened to the first hollow body (4) and/or the second hollow body (5).

13. An apparatus according to one of the claims 1 to 12, **characterized in that** the first hollow body (4) and the second hollow body (5) can be inflated separately from each other.

## Revendications

1. Dispositif pour disposer de façon prédéterminée un extenseur (2) dans un vaisseau sanguin (3), lequel dispositif présente un premier corps creux gonflable (4) et un deuxième corps creux gonflable (5), le premier corps creux (4) et le deuxième corps creux (5) ayant chacun, dans l'état dégonflé des deux corps creux (4, 5), la forme d'un cylindre, et une extension longitudinale du premier corps creux (4) étant sensiblement alignée sur une extension longitudinale du deuxième corps creux (5), une face frontale (6) du deuxième corps creux (5) étant contiguë à une face frontale du premier corps creux (4) dans l'état dégonflé, **caractérisé en ce que** le dispositif est conformé de telle façon qu'il change de forme de façon prédéterminée lors du gonflage, **en ce que**, dans un état gonflé des deux corps creux (4, 5), le premier corps creux (4) et le deuxième corps creux (5) ont chacun sensiblement la forme d'un cylindre, **en ce que** l'extension longitudinale du deuxième corps creux (5) forme un angle pouvant être prédéterminé de 20° à 160° par rapport à l'extension longitudinale du premier corps creux (4) et **en ce que**, dans l'état gonflé, le deuxième corps creux (5) est disposé sur une surface d'enveloppe du premier corps creux (4).

2. Dispositif selon la revendication 1, **caractérisé en ce que** le deuxième corps creux (5) est disposé pour l'essentiel de façon sensiblement contiguë au premier corps creux (4).

3. Dispositif selon la revendication 1 ou 2, **caractérisé en ce que**, dans l'état gonflé, le deuxième corps creux (5) est entouré des deux côtés, dans l'extension longitudinale du premier corps creux (4), par la surface d'enveloppe du premier corps creux (4).

4. Dispositif selon l'une des revendications 1 à 3, **caractérisé en ce qu'**une paroi (12) du premier corps creux (4) se compose d'au moins une première zone (7) et une deuxième zone (8), et **en ce que** la première zone (7) et la deuxième zone (8) ont une extensibilité différente.

5. Dispositif selon la revendication 4, **caractérisé en ce que** la première zone (7) et la deuxième zone (8) présentent des épaisseurs de paroi différentes ou **en ce que** la première zone (7) et la deuxième zone (8) ont une élasticité différente.

6. Dispositif selon l'une des revendications 4 ou 5, **caractérisée en ce que** dans l'état gonflé, la première zone (7) est disposée entre une extrémité proximale (10) du premier corps creux (4) et le deuxième corps creux (5) et **en ce que** la deuxième zone (8) est plus extensible que la première zone (7).

7. Dispositif selon l'une des revendications 1 à 6, **caractérisé en ce qu'**un grand nombre pouvant être prédéterminé de fils (11) reliés à une paroi (12) du premier corps creux (4) sont disposés à l'intérieur du premier corps creux (4) et **en ce que** les fils (11) sont tendus dans l'état gonflé pour définir la forme du premier corps creux (4).

8. Dispositif selon la revendication 7, **caractérisé en ce qu'**un nombre pouvant être prédéterminé des fils (11) sont fixés chacun à une partie (13) de la paroi (12) du premier corps creux (4) contiguë au deuxième corps creux (5).

9. Dispositif selon l'une des revendications 1 à 8, **caractérisé en ce que** des éléments de soutien (14) sont disposés sur une paroi (12) du premier corps creux (4) et **en ce que** les éléments de soutien (14) définissent, dans l'état gonflé, la forme du premier corps creux (4).

10. Dispositif selon la revendication 9, **caractérisé en ce que** les éléments de soutien (14) sont sensiblement elliptiques dans l'état dégonflé et sensiblement circulaires dans l'état gonflé.

11. Dispositif selon la revendication 9 ou 10, **caractérisé en ce que** les éléments de soutien (14) sont affectés à un premier groupe (15) ou à un deuxième groupe (16) et **en ce que**, dans l'état gonflé, une partie (13) de la paroi (12) du premier corps creux (4) contiguë au corps creux (5) est disposée entre le premier groupe (15) et le deuxième groupe (16).

12. Dispositif selon l'une des revendications 1 à 11, **caractérisé en ce que** le premier corps creux (4) est disposé, dans l'état dégonflé, dans une gaine de transport (17) sensiblement cylindrique, qui est de préférence fixée sur le premier corps creux (4) et/ou le deuxième corps creux (5).

13. Dispositif selon l'une des revendications 1 à 12, **caractérisé en ce que** le premier corps creux (4) et le deuxième corps creux (5) peuvent être gonflés séparément l'un de l'autre.
